# EUROPÄISCHE PATENTANMELDUNG

(11) **EP 1 155 707 A1**
(43) Veröffentlichungstag der Anmeldung: **21.11.2001**
(21) Anmeldenummer: 01109985.0
(22) Anmeldetag: 25.04.2001
(51) Int. Cl.: A61M 5/14

(54) **Infusionspumpe**

(30) Priorität: 18.05.2000 DE 20008961 U
(71) Anmelder: B. BRAUN MELSUNGEN AG, 34212 Melsungen (DE)
(72) Erfinder: Angersbach, Klaus, Dipl.-Ökonom, 34326 Morschen (DE); Heitmeier, Rolf, Dipl.-Ing., 34225 Baunatal (DE)
(74) Vertreter: Selting, Günther, Dipl.-Ing.

(57) **Zusammenfassung**

Die Infusionspumpe besteht aus einem mobilen, selbstständig funktionsfähigen Basismodul (10), das einen Pumpenteil und einen Energiespeicher enthält. Für anspruchsvolle Programmier- und Leistungsaufgaben wird das Basismodul (10) in ein Bedienmodul (20) eingesetzt, welches eine Eingabevorrichtung (27) für komplexe Eingaben aufweist. Damit kann die Infusionspumpe als Basismodul (10) oder als kombiniertes Basismodul mit Bedienmodul (20) eingesetzt werden.

## Beschreibung

Die Erfindung betrifft eine Infusionspumpe zur Verabreichung von Flüssigkeit zu einem Patienten.

Infusionspumpen werden zur intravenösen Verabreichung von Medikamenten, zur künstlichen enteralen oder parenteralen Ernährung und zur Schmerztherapie eingesetzt. Hierbei ergeben sich zwei typische Einsatzgebiete, nämlich der stationäre Einsatz und der mobile Einsatz. Für beide Einsatzgebiete gibt es spezielle Infusionspumpen. Für den mobilen Einsatz wurden mobile Infusionspumpen entwickelt, die relativ klein und leichtgewichtig ausgeführt sind, jedoch ein relativ begrenztes Leistungsspektrum haben, was die Programmierbarkeit und die Bedienungsmöglichkeiten angeht. Ferner gibt es stationäre Infusionspumpen, die umfassende Bedienungsmöglichkeiten haben und ohne jegliche zeitliche Begrenzung am elektrischen Versorgungsnetz betrieben werden können.

Der Erfindung liegt die Aufgabe zu Grunde eine Infusionspumpe zu schaffen, die sowohl für den stationären Einsatz als auch für den mobilen Einsatz geeignet ist und hierbei jeweils diejenigen Eigenschaften hat, die für das betreffende Einsatzgebiet gefordert werden.

Die Lösung dieser Aufgabe erfolgt erfindungsgemäß mit den im Anspruch 1 angegebenen Merkmalen. Hiernach besteht die Infusionspumpe aus einem Basismodul, das eine selbstständige mobile Pumpe bildet und einem Bedienmodul mit welchem das Basismodul wahlweise kombiniert werden kann, um eine stationäre Infusionspumpe mit erweitertem Leistungsspektrum zu bilden. Das Bedienmodul stellt für sich genommen keine funktionsfähige Infusionspumpe dar. Es enthält Zusatzkomponenten für eine Infusionspumpe, wie eine Recheneinheit, eine Speichereinheit, eine Datenbank, ein Diskettenlaufwerk oder eine ähnliche Wiedergabevorrichtung für Datenträger und insbesondere eine Eingabevorrichtung, die eine komplexe und vielseitige Eingabe von Infusionsparametern ermöglicht. Vorzugsweise ist das Bedienmodul mit einer Anzeigevorrichtung in Form eines Bildschirms versehen, wobei auf dem Bildschirm einerseits alphanumerische Angaben angezeigt werden, andererseits aber auch graphische Anzeigen, wie beispielsweise ein Zeitprofil der Infusionsrate. Die Basismodule sind standardisierte Pumpenmodule, die einen Pumpenteil mit zugehörigem Antrieb aufweisen und für sich genommen funktionsfähig sind, jedoch begrenzte Programmier- und Laufzeitkapazitäten haben. Zusätzlich ist das Bedienmodul vorgesehen, das eine universellere Programmierung bei erhöhtem Bedienungskomfort ermöglicht.

Die erfindungsgemäße Infusionspumpe ist in unterschiedlichen Stadien einer Krankheit oder medizinischen Behandlung einsetzbar. Sie erlaubt den Übergang eines chronisch kranken Patienten in die häusliche Pflege, erleichtert den Mobilisierungsprozess des Patienten im Krankenhaus, gestattet innerklinische Transportprozesse und ist auch für eine intensivmedizinische Maximalversorgung geeignet. Die Pumpe ist universell einsetzbar, so dass für die unterschiedlichen Anwendungen keine spezifischen Pumpen erforderlich sind. Hierzu gehört auch, dass der Patient bei unterschiedlichen Anwendungen mit dem selbem Schlauchsystem verbunden bleibt, wobei es sich in der Regel um Einmalschläuche handelt.

Das Basismodul hat eine minimierte Benutzeroberfläche und eine eigene Energieversorgung und kann über Verbindungselemente für den Anschluss zusätzlicher Bedienelemente verfügen. Ferner weist das Basismodul die zur intravenösen Anwendung erforderlichen Sicherheitsvorkehrungen auf. Die minimierte Benutzeroberfläche bedeutet beispielsweise, dass ein alphanumerisches Display und maximal fünf Bedienelemente vorhanden sind. Die Bedienelemente können durch das Display in ihrer Funktion beschriftete Tasten sein (Softkeys) oder mechanische Schalter oder andere Bedieneinrichtungen, die auf unterschiedliche Betätigungen unterschiedlich reagieren. Grundsätzlich gehört zu der Einstellvorrichtung eine Auswahlvorrichtung, die das Hochschalten oder Herunterschalten einer Liste auszuwählender Betriebsrahmen ermöglicht, eine Quittiereinrichtung sowie eine Verstelleinrichtung für das Erhöhen oder Erniedrigen von Zahlenwerten.

Die datentechnische und mechanische Kopplung des Basismoduls mit dem Bedienmodul dient dem Datenaustausch und der mechanischen Fixierung des Basismoduls. Der Datenaustausch kann elektrisch, elektromagnetisch, z. B. über Hochfrequenz, optisch, z. B. per Infrarotschnittstelle, per Schall, z. B. Ultraschall, oder mittels Datenträger erfolgen. Zur mechanischen Fixierung dienen mechanische Verbindungselemente. Diese können so ausgeführt sein, dass die Fixierung durch eine Führung mit Verriegelungsmechanismus ausgeführt ist und integraler Bestandteil des Bedienmoduls ist.

Das Basismodul verfügt über einen eigenen Energiespeicher, der den eigenständigen Betrieb für die Nutzungsdauer innerhalb der Mobilitätsphase sicherstellt. Dieser Energiespeicher kann von außen austauschbar sein oder fest integriert sein. Die Überwachung der verfügbaren Kapazität und deren Visualisierung wird vom Basismodul wahrgenommen. Die Aufladung des Energiespeichers kann durch das Bedienmodul erfolgen oder durch ein zusätzliches netzgespeistes Ladegerät. Wird das Basismodul mit dem Bedienmodul verbunden, übernimmt das Bedienmodul automatisch die Energieversorgung.

Das Basismodul besitzt eine Schnittstelle zu einer Fernbedienung. Diese kann beispielsweise einen Patiententaster zur Auslösung von Schmerzmittelboli sein. Die Fernbedienung kann drahtlos oder drahtgebunden mit dem Basismodul kommunizieren.

Im Folgenden wird ein Ausführungsbeispiel der Erfindung, unter Bezugnahme auf die Zeichnungen näher erläutert.

Es zeigen:
Figur 1 eine perspektivische Darstellung einer Infusionspumpe, die aus einem Basismodul und einem Bedienmodul besteht,
Figur 2 eine isolierte Darstellung des Basismoduls,
Figur 3 das Einsetzen des Basismoduls in das Bedienmodul bei einer ersten Orientierung des Basismoduls und
Figur 4 das Einsetzen des Basismoduls in das Bedienmodul bei einer zweiten Orientierung des Basismoduls.

Die Infusionspumpe weist ein Basismodul 10 auf, das eine voll funktionsfähige Mobilinfusionspumpe bildet. Das Basismodul 10 besitzt ein Gehäuse 11, welches beispielsweise eine Spritzenpumpe oder Rollenpumpe und eine (nicht dargestellte) Antriebsvorrichtung und einen Energiespeicher in Form einer elektrischen Batterie enthält. Außerdem enthält das Gehäuse eine Steuereinrichtung in Form eines Mikroprozessors. Zu dem Gehäuse 11 führt ein Zuleitungsschlauch 12, der von einem Infusionsbehältnis kommt. Von dem Gehäuse weg führt ein Patientenschlauch 13, an den der Patient angeschlossen ist.

An der Frontseite des Gehäuses 10 befindet sich eine Benutzeroberfläche 14, die ein Display 15, mit einer Einstellvorrichtung 16, einer Auswahlvorrichtung 17 sowie einer Quittiertaste 18 aufweist. Die Benutzeroberfläche 14 dient zur Anzeige und zum Einstellen von Pumpenparametern.

Das Basismodul 10 verfügt über die für eine intravenöse Therapie erforderlichen Sicherheitseinrichtungen, nämlich Maßnahmen gegen ungewollte Luftinfusion, Maßnahmen gegen Über- oder Unterdosierung, Maßnahmen gegen zu hohen Druckaufbau im Patientenschlauch und Maßnahmen gegen Verschluss der Strecke zwischen dem Infusionsbehälter und dem Pumpenmodul. Das Pumpenmodul 10 ist ein mobiles Modul, das am Patientenkörper, am Patientenbett, oder an einer anderen mobilen Einrichtung befestigt werden kann und ohne ortsfesten Anschluss mitgeführt wird. Bei Bedarf kann das Basismodul in eine Ladestation eingesetzt werden, um den Energiespeicher aufzuladen.

Das Basismodul 10 kann in ein hierfür vorgesehenes Bedienmodul 20 eingesetzt werden. Das Bedienmodul 20 weist ein langgestrecktes Gehäuseteil 21 mit L-förmigem Profil auf, das eine Bodenwand 22 und ein rückwärtiges Gehäuseteil 23 bildet. Am vorderen Ende der Bodenwand 22 ist ein Frontgehäuse 24 befestigt, das um eine Achse 25 herumgeklappt werden. Im aufgerichteten Zustand bildet das Frontgehäuse 24 mit dem L-förmigen Gehäuse 21 eine U-förmige oder rinnenförmige Struktur, die das Basismodul 10 passend umschließt. Das Frontgehäuse 24 bedeckt dabei die Vorderseite des Basismoduls.

Das Frontgehäuse 24 weist eine Anzeigevorrichtung 26 in Form eines Bildschirms zum Anzeigen graphischer Bilddarstellungen und alphanumerischer Angaben auf sowie eine Eingabevorrichtung 27 mit alphanumerischer Tastatur 28 und Bedientasten 29. Das Bedienmodul wird beim Einsetzen des Basismoduls funktionell mit dem Basismodul verbunden, so dass das entstehende Gesamtgerät eine hochgerüstete Funktionspumpe bildet. Das Bedienmodul hat gegenüber dem Basismodul eine erweiterte Benutzeroberfläche sowie zusätzliche Sensorenanschlüsse (nicht dargestellt) und zusätzliche Alarmierungsanschlüsse. Es enthält eine erweitere Datenbank für Medikamente sowie eine datentechnische Kopplung mit einem zentralen Datenerfassungssystem. Das Bedienmodul ist mit einer Halterung zur Anbringung an einer Wandschiene oder einem Infusionsständer 29 versehen. Außerdem kann es eine Wiedergabevorrichtung zum Lesen von Datenträgern aufweisen.

Das Bedienmodul 20 erlaubt eine variierbare Einstellung des Basismoduls. Es kann als Programmiereinheit für das Basismodul benutzt werden. Im Bedienmodul werden komplexere Funktionen visualisiert und bedient. Generell stellt das Bedienmodul eine Leistungserweiterung für die Bedienung dar, während die eigentliche Durchführung der parametrisierten Therapie durch das Basismodul erfolgt und auch bei Trennung vom Bedienmodul weiterhin vom Basismodul abgearbeitet wird.

Das Bedienmodul kann mechanisch mit weiteren gleichartigen Bedienmodulen gekoppelt werden, so dass eine Einheit aus mehreren Bedienmodulen entsteht.

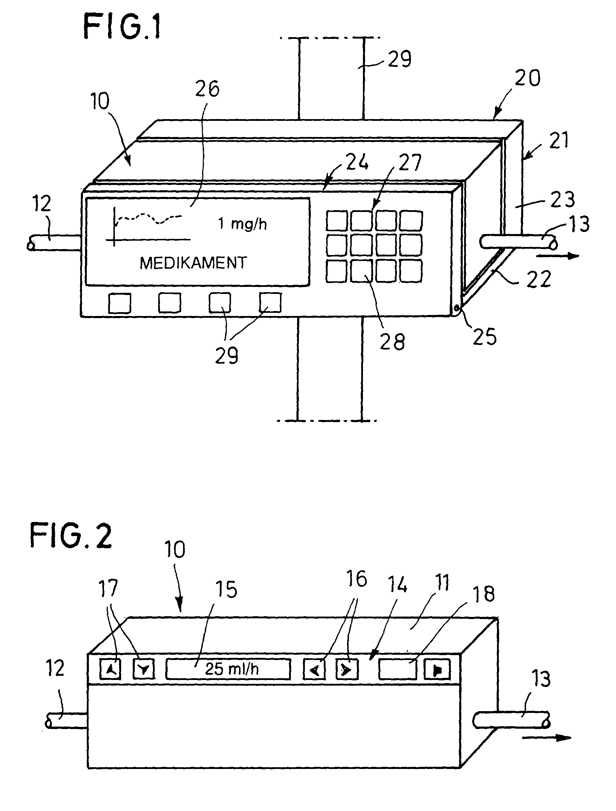

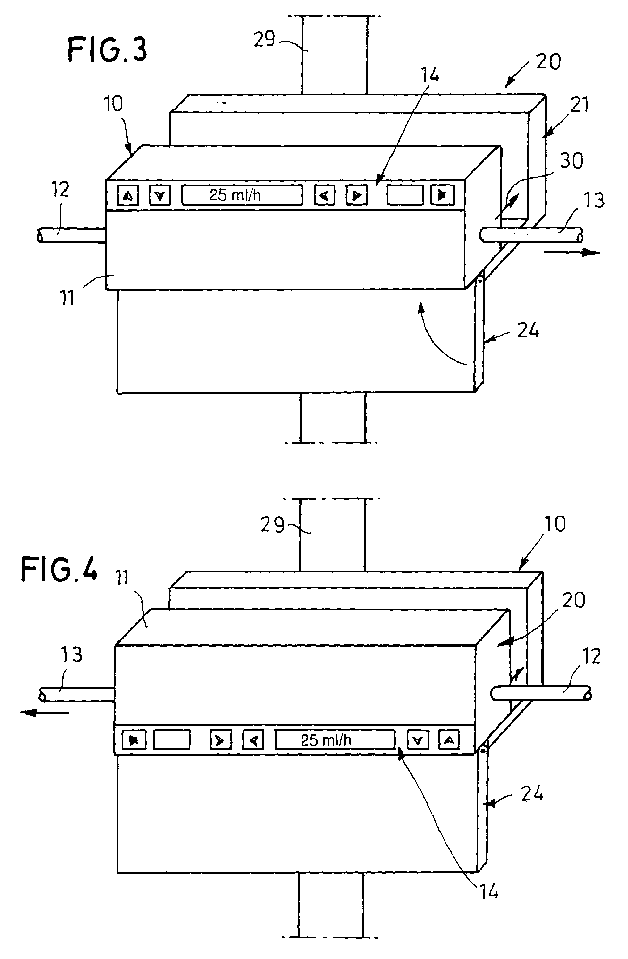

Figur 3 zeigt das Einsetzen des Pumpenmoduls in das Bedienmodul. Hierbei ist das Frontgehäuse des Bedienmoduls 24 aufgeklappt. Das Basismodul wird in Richtung des Pfeils 30 in das Bedienmodul eingeschoben, wobei automatisch die elektrischen Verbindungen hergestellt werden und eine mechanische Kopplung erfolgt. Sodann wird das Frontgehäuse 24 hochgeklappt, so dass der in Figur 1 dargestellte Zustand entsteht. In diesem Zustand verdeckt das Frontgehäuse mit seiner Anzeige- und Eingabevorrichtung die Bedienoberfläche des Basismoduls, so dass der Benutzer nicht versehentlich an dem Basismodul Einstellungen vornehmen kann. Wenn das Basismodul in das Bedienmodul eingesetzt ist, erfolgen sämtliche Einstellungen und Anzeigen ausschließlich am Bedienmodul.

Bei dem Basismodul 10 geht der Patientenschlauch 13 in der Darstellung nach den Figuren 1 bis 3 vom rechten Ende des Gehäuses 11 ab. In Figur 4 ist derselbe Zustand wie in Figur 3 dargestellt, jedoch ist das Basismodul 20 derart umgesetzt, dass das Ende, von dem der Patientenschlauch 13 ausgeht nunmehr nach links zeigt. Die Benutzeroberfläche 14, die gemäß Figur 3 am oberen Ende des Gehäuses angeordnet war, ist in Figur 4 am unteren Ende der Frontseite angeordnet. Das Gehäuse 11 ist somit in Figur 4 gegenüber dem Zustand von Figur 3 um 180° gedreht und die Anzeigevorrichtungen der Benutzeroberfläche 14 werden auf dem Kopf stehend wiedergegeben, derart dass die Anzeigevorrichtungen bei unterschiedlichen Orientierungen des Gehäuses 11 lesegerecht ablesbar sind. Die Schlauchführung kann somit entsprechend der Position des Patienten wahlweise derart geändert werden, dass der Patientenschlauch 13 in unterschiedlichen Richtungen abgeht.

## Patentansprüche

1. Infusionspumpe mit einem selbstständig funktionsfähigen mobilen Basismodul (10), das einen Pumpenteil, einen Energiespeicher und eine Einstellvorrichtung (16) aufweist und ein Bedienmodul (20), welches an das Basismodul (10) zur Energieübertragung und zur Datenübertragung ankoppelbar ist und das eine Eingabevorrichtung (17) aufweist und zusammen mit dem Basismodul eine hochgerüstete Pumpeneinheit bildet.

2. Infusionspumpe nach Anspruch 1, **dadurch gekennzeichnet, dass** das Bedienmodul (20) Sensoranschlüsse aufweist.

3. Infusionspumpe nach Anspruch 1 oder 2, **dadurch gekennzeichnet, dass** das Bedienmodul (20) mindestens einen Alarmierungsanschluss aufweist.

4. Infusionspumpe nach einem der Ansprüche 1 bis 3, **dadurch gekennzeichnet, dass** das Bedienmodul (20) eine Datenbank für Medikamente enthält.

5. Infusionspumpe nach einem der Ansprüche 1 bis 4, **dadurch gekennzeichnet, dass** das Bedienmodul (20) eine Halterung für eine Installation an Trageinrichtungen (29) aufweist.

6. Infusionspumpe nach einem der Ansprüche 1 bis 5, **dadurch gekennzeichnet, dass** das Bedienmodul (20) eine Wiedergabevorrichtung zum Lesen von Datenträgern enthält.

7. Infusionspumpe nach einem der Ansprüche 1 bis 6, **dadurch gekennzeichnet, dass** das Basismodul (10) an einem langgestreckten Gehäuse (11) eine Anzeigevorrichtung (15) aufweist, deren Anzeige auf dem Kopf stehend wiedergebbar ist, derart, dass sie bei unterschiedlichen Orientierungen des Gehäuses (11) lesegerecht ablesbar ist.

8. Infusionspumpe nach einem der Ansprüche 1 bis 7, **dadurch gekennzeichnet, dass** das Bedienmodul (20) eine Programmierstation für das Basismodul (10) bildet.

9. Infusionspumpe nach einem der Ansprüche 1 bis 8, **dadurch gekennzeichnet, dass** das Bedienmodul (20) eine Aufnahme aufweist, in die das Basismodul (10) einsetzbar ist und die die Einstellvorrichtung (16) des Basismoduls (10) bedeckt.
